## Europäisches Patentamt

## European Patent Office

## Office européen des brevets

(11) Veröffentlichungsnummer: **0 189 756**
**B1**

# EUROPÄISCHE PATENTSCHRIFT

(45) Veröffentlichungstag der Patentschrift:
14.12.88

(51) Int. Cl.⁴: **A 61 B 17/22, G 10 K 11/28**

(21) Anmeldenummer: **86100167.5**

(22) Anmeldetag: **08.01.86**

(54) Einrichtung zur Erzeugung zeitlich versetzter Stosswellen.

(30) Priorität: **21.01.85 DE 3501838**

(43) Veröffentlichungstag der Anmeldung:
**06.08.86 Patentblatt 86/32**

(45) Bekanntmachung des Hinweises auf die Patenterteilung:
**14.12.88 Patentblatt 88/50**

(84) Benannte Vertragsstaaten:
**DE FR GB NL**

(56) Entgegenhaltungen:
**DE-C-562 266
DE-C-760 163
US-A-3 451 260**

(73) Patentinhaber: **Siemens Aktiengesellschaft Berlin und München, Wittelsbacherplatz 2, D-8000 München 2 (DE)**

(72) Erfinder: **Reichenberger, Helmut, Dr., Begonienstrasse 28, D-8501 Eckental (DE)**

EP 0 189 756 B1

**Beschreibung**

Die Erfindung betrifft eine Einrichtung zur Erzeugung einer akustischen Stoßwelle und zur Fokussierung derselben auf einen Fokusbereich mit einem einzigen Stoßwellenrohr. Sie bezieht sich insbesondere auf die Zertrümmerung von Konkrementen in einem Lebewesen.

Stoßwellenrohre zur Erzeugung akustischer Stoßwellen sind an sich seit längerer Zeit bekannt. Sie können nach neueren Untersuchungen, wie z. B in der DE-A-3 312 014 angegeben, in der Medizintechnik zur Zertrümmerung von Konkrementen im Körper eines Patienten eingesetzt werden. In der DE-A-3 312 014 ist eine Einrichtung der eingangs genannten Art beschrieben. Es handelt sich dabei um ein Stoßwellenrohr, dessen Spule eine gekrümmte Form aufweist, so daß die ausgesandte Stoßwelle in einem Fokus zusammenläuft. Vor der Spule sind eine Isolierfolie und eine Metallmembran angeordnet. Auf die Spule wird mittels eines Kondensators ein Spannungsimpuls gegeben, demzufolge die Metallmembran von der Spule abgestoßen wird. Auf diese Weise entsteht die für eine medizinische Behandlung nötige Stoßwelle.

Vor dem Auslösen der nächsten Stoßwelle muß der Kondensator wieder aufgeladen werden, wozu eine gewisse Zeit benötigt wird.

Zur Zertrümmerung eines Konkrements, z. B. eines Nierensteins in einem erwachsenen Menschen, ist eine Vielzahl (z. B. 500) solcher Stoßwellen und damit eine entsprechende Behandlungsdauer nötig. Es ist auch zu erwarten, daß die Wirkung der Stoßwellen verbessert werden kann, wenn die Stoßwellen so kurzzeitig hintereinander auf das Konkrement auftreffen, daß sie sich in ihrer Wirkung gegenseitig überschneiden. Bezugnehmend auf diese Gedanken wurde bereits in der DE-A-3 328 039 vorgeschlagen, mehrere Stoßwellenrohre parallel anzuordnen und diese kurz nacheinander in einer vorgegebenen Reihenfolge zu aktivieren. Diese vorgehensweise verursacht aber einen verhältnismäßig hohen Kostenaufwand, da wesentliche Teile der Anordnung wie z. B. Kondensator, Funkenstrecke und das Stoßwellenrohr selber, mehrfach aufgebaut sein müssen.

Aufgabe vorliegender Erfindung ist es, eine Einrichtung der eingangs genannten Art so weiterzubilden, daß trotz Vorhandenseins nur eines einzigen Stoßwellenrohrs zeitlich versetzte Stoßwellen auf das Konkrement einwirken können.

Die Erfindung geht aus von der Überlegung, daß auf weitere Stoßwellenrohre verzichtet werden kann, wenn es gelingt, die von dem einen Stoßwellenrohr abgegebene Stoßwelle in eine Mehrzahl einzelner, kleinerer Stoßwellen mit geringerer Intensität aufzulösen und diese gegeneinander verzögert auf das in einem Fokusbereich befindliche Konkrement einwirken zu lassen.

Die genannte Aufgabe wird erfindungsgemäß dadurch gelöst, daß das Stoßwellenrohr zur Erzeugung einer ebenen Stoßwelle ausgebildet ist, daß eine Teilvorrichtung für die Stoßwelle vorgesehen ist, die die Stoßwelle in Richtung auf einen ersten und einen zweiten Reflektor teilt, daß der erste und der zweite Reflektor einen ersten bzw. zweiten Brennpunkt besitzen, die in bezug auf die Teilvorrichtung so angeordnet sind, daß sie gemeinsam in dem Fokusbereich liegen, und daß der erste und der zweite Reflektor von der Teilvorrichtung ungleich weit entfernt sind.

Vorteil dieser Einrichtung ist es, daß mit einer vom Stoßwellenrohr ausgehenden Stoßwelle am Konkrement zwei zeitlich aufeinander folgende Stoßwellen wirksam werden, damit die Anzahl der auszulösenden Stoßwellen halbiert wird und somit eine erhebliche Verkürzung der Behandlungsdauer erreicht wird. Dadurch wird die Belastung für den Patienten reduziert sowie die Nutzbarkeit der Anlage und ihre Wirtschaftlichkeit erhöht.

Ein weiterer Vorteil dieser Einrichtung ist es, daß bei geeigneter Dimensionierung zwei Stoßwellen zeitlich so versetzt auf das Konkrement eintreffen können, daß sich ihre Wirkungen überlagern. Dabei ist lediglich ein einziges Stoßwellenrohr erforderlich, wodurch die Einrichtung kompakt und wirtschaftlich aufgebaut werden kann.

Ein weiterer Vorteil ergibt sich, wenn eine Mehrzahl an ersten oder zweiten Reflektoren zur Verfügung steht, die eine unterschiedliche Krümmung aufweisen und austauschbar an der Einrichtung befestigt sind. Dann kann durch Auswechseln des austauschbaren Reflektors und Ersatz durch einen anderen die Größe der Zeitversetzung der beiden Stoßwellen im Konkrement eingestellt werden. Ein solches Auswechseln kann durch die Größe oder Art des zu zerstörenden Konkrements bedingt sein.

Weitere Vorteile und Ausgestaltungen der Erfindung ergeben sich aus der folgenden Beschreibung eines Ausführungsbeispiels anhand einer Figur in Verbindung mit den Unteransprüchen.

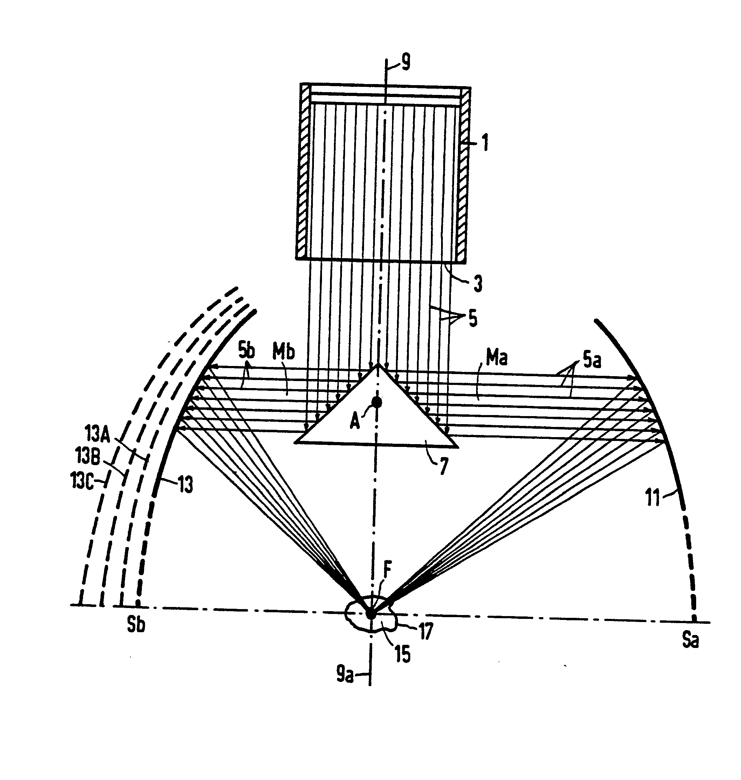

Die Figur zeigt ein Stoßwellenrohr mit einem ersten und zweiten Reflektor in ungleichen Abständen zu einer Teilvorrichtung.

In der Figur ist mit 1 ein an sich bekanntes Stoßwellenrohr bezeichnet, an dessen Austrittsfenster 3 nach Aktivierung eine Stoßwelle 5 austritt. Die Stoßwelle 5 ist hier infolge des gegebenen Aufbaus des Stoßwellenrohrs 1 eine ebene Welle, die auf eine Teilvorrichtung 7 auftrifft. Die Teilvorrichtung 7 besteht aus einem Ultraschallwellen reflektierenden Material. Sie ist ein vorzugsweise aus Messing gefertigter Kegel, insbesondere ein 90°-Kegel, dessen Spitze in Richtung auf das Stoßwellenrohr 1 zeigt. Es kommen auch andere Kegelformen in Frage. Die Stoßwellenrohrachse 9 fällt mit der Rotationsachse 9a des Kegels zusammen.

Von der kegelförmigen Stoßwellen-Teilvorrichtung 7 wird die Stoßwelle 5 rechtwinklig reflektiert, so daß sie senkrecht zur Stoßwellenrohrachse 9, d. h. radial auseinanderläuft. Der in der Schnittdarstellung nach rechts reflektierte Teil 5a der Stoßwelle 5 trifft auf einen ersten gekrümmten Reflektor 11. Der nach links reflektierte Teil 5b der Stoßwelle 5 trifft auf einen zweiten, anders gekrümmten Reflektor 13. Der erste Reflektor 11 und der zweite Reflektor 13 sind so positioniert, daß sie ungleich weit (Ma > Mb) von dem Kegel 7 entfernt sind und gleichzeitig ihren jeweiligen Brennpunkt in einem gemeinsamen Punkt F haben. In diesem Punkt F ist ein Konkrement 15 plaziert, welches zertrümmert werden soll. Der Punkt F ist somit der Fokusbereich der dargestellten Einrichtung. Anstelle von zwei halbkreisförmigen Reflektoren 11, 13 können auch vier sektorförmige Reflektoren angeordnet werden, deren Krümmung oder Parabelparameter unterschiedlich sind oder aber bei jeweils gegenüber liegenden Sektoren auch gleich sein können.

Aus der Figur ist zu entnehmen, daß der erste und der zweite Reflektor 11, 13 jeweils Teil eines Ringes sind, der durch Rotation des Bogens einer ersten bzw. einer zweiten Parabel, deren Scheitelpunkt in Sa bzw. Sb und deren Brennpunkt jeweils in F liegt, um die Achse des Kegels 7 gebildet ist, und daß die beiden Reflektoren 11, 13 an gegenüberliegenden Seiten der Achse 9a des Kegels 7 angeordnet sind.

Aufgrund des größeren Abstands Ma des ersten Reflektors 11 zum Kegel 7 im Vergleich zu dem Abstand Mb des zweiten Reflektors 13 zum Kegel 7 legt der rechte Teil Sa der Stoßwelle 5 einen längeren Weg zurück, bis er zum gemeinsamen Punkt F gelangt, als der linke Teil 5b der Stoßwelle 5. Dadurch trifft der rechte Teil 5a der Stoßwelle 5 mit einer Zeitverzögerung gegenüber dem linken Teil 5b in dem Punkt F ein. Dieser Zeitversatz sollte vorzugsweise so bemessen sein, daß die rechte Teilwelle 5a in dem Konkrement 15 einläuft, wenn die linke Teilwelle 5b bereits am hinteren Rand 17 des Konkrements 15 reflektiert wurde und als Zugwelle durch das Konkrement 15 zurückläuft.

Die Abstände Ma, Mb sind vorliegend auf die Mittellinien der Teilwellen 5a, 5b bezogen. Bei einem Parabelparameter des ersten Reflektors 11 von z. B. $p_1 = 24$ cm und des zweiten Reflektors 13 von z. B. $p_2 = 21,5$ cm und einem Abstand des Kegelmittelpunktes A vom Punkt F von 10 cm ergibt sich eine Wegdifferenz für die beiden Teilwellen 5a, 5b von ca. 1,5 cm. Diese Differenz entspricht einer Zeitverzögerung von ca. 10 µs und ist gut geeignet, die Überlagerung der Wirkung beider Teilwellen 5a, 5b zu erzielen

Um den Zeitversatz variieren zu können, steht eine ganze Anzahl zweiter Reflektoren 13, 13A, 13B, 13C zur Verfügung, von denen jeder einzelne eine andere parabolische Krümmung aufweist Diese zweiten Reflektoren 13, 13A, 13B, 13C müssen dann jeweils in einem etwas anderem Abstand Mb zum Kegel 7 angeordnet werden, um ihren Brennpunkt in dem gemeinsamen Punkt F zu haben. Durch Austausch ergibt sich eine veränderte Laufzeit der Teilwelle 5b, wodurch die Zeitdifferenz zwischen der rechten Teilwelle 5a und der linken Teilwelle 5b einstellbar ist. Die gerade nicht benötigten Reflektoren 13, 13A, 13B, 13C werden entfernt oder ausgeklappt.

**Patentansprüche**

1. Einrichtung zur Erzeugung einer akustischen Stoßwelle und zur Fokussierung derselben auf einen Fokusbereich mit einem einzigen Stoßwellenrohr, dadurch gekennzeichnet, daß das Stoßwellenrohr (1) zur Erzeugung einer ebenen Stoßwelle (5) ausgebildet ist, daß eine Teilvorrichtung (7) für die Stoßwelle (5) vorgesehen ist, die die Stoßwelle (5) in Richtung auf einen ersten und einen zweiten Reflektor (11, 13) teilt, daß der erste und der zweite Reflektor (11, 13) einen ersten bzw. zweiten Brennpunkt besitzen, die in bezug auf die Teilvorrichtung (7) so angeordnet sind, daß sie gemeinsam in dem Fokusbereich (F) liegen, und daß der erste und der zweite Reflektor (11, 13) von der Teilvorrichtung (7) ungleich weit entfernt sind.

2. Einrichtung nach Anspruch 1, dadurch gekennzeichnet, daß als Teilvorrichtung (7) ein Kegel aus einem reflektierenden Material vorgesehen ist, der die Stoßwelle (5) auf den ersten und den zweiten Reflektor (11, 13) verteilt.

3. Einrichtung nach Anspruch 2, dadurch gekennzeichnet, daß der Kegel ein 90°-Kegel ist, dessen Spitze zum Stoßwellenrohr (1) weist.

4. Einrichtung nach Anspruch 2 oder 3, dadurch gekennzeichnet, daß der erste und der zweite Reflektor (11, 13) jeweils Teil eines Ringes sind, der durch Rotation des Bogens einer ersten bzw. einer zweiten Parabel um die Achse des Kegels (7) gebildet ist, und daß die beiden Reflektoren (11, 13) an gegenüberliegenden Seiten der Achse (9a) des Kegels (7) angeordnet sind.

5. Einrichtung nach einem der Ansprüche 2 bis 4, dadurch gekennzeichnet, daß der Kegel (7) aus Messing besteht.

6. Einrichtung nach einem der Ansprüche 2 bis 5, dadurch gekennzeichnet, daß der eine der beiden Reflektoren (11, 13) austauschbar ist.

7. Einrichtung zur Erzeugung einer akustischen Stoßwelle und zur Fokussierung derselben auf einen Fokusbereich mit einem einzigen Stoßwellenrohr, dadurch gekennzeichnet, daß die von dem Stoßwellenrohr (1) abgegebene Stoßwelle (5) in eine Mehrzahl einzelner Stoßwellen (5a, 5b) geteilt ist, die unterschiedliche Laufzeiten haben.

**Claims**

1. A device for the production of an acoustic shock wave and for focussing it on a focal region with a single chock-wave tube, characterized in that the shock wave tube (1) is conctructed to produce a flat chock wave (5), that a splitting device (7) is provided for the shock wave (5) which divided the shock wave (5) between a first and a second reflector (11, 13), that the first and second reflector (11, 13) have recpectively a first and a second focal point which, in relation to the splitting device (7), are arranged so that they lie together in the focal region (F), and that the first and second reflectors (11, 13) are at different distances from the splitting device (7).

2. A device according to claim 1, characterised in that, as the splitting device (7), a cone of a reflective material is provided which distributes the chock wave (5) over the first and second reflectors (11, 13).

3. A device according to claim 2, characterized in that the cone is a 90° cone, the tip of which points to the chock wave tube (1).

4. A device according to claim 2 or claim 3, characterised in that the first and second reflectors (11, 13) are respectively part of a ring formed by the rotation of the arch of respective first and second parabolas about the axis of the cone (7), and that the two reflectors (11, 13) are arranged on opposite sides of the axis (9a) of the cone (7).

5. A device according to any one of claims 2 to 4, characterized in that the cone (7) consits of brass.

6. A device according to any one of claims 2 to 5, characterised in that one of the two reflectors (11, 13) is exchangeable.

7. A device for the production of an acoustic shock wave and for focussing it on a focal region with a single shock wave tube, characterised in that the shock wave (5) generated by the shock wave tube (1) is divided into a plurality of individual shock waves (5a, 5b) which are have different travel times.

**Revendications**

1. Dispositif pour produire une onde de choc acoustique et pour la focalisation de celle-ci sur une zone focale, avec un tube de choc unique, caractérisé par le fait que le tube de choc (1) est réalisé pour produire une onde de choc plane, qu'il est prévu un dispositif diviseur (7) pour l'onde de choc (5), qui divise l'onde de choc (5) en direction d'un premier et d'un second réflecteurs (11, 13), et que le premier et le second réflecteurs (11, 13) possèdent un premier et un second foyers qui sont disposés de telle façon par rapport au dispositif diviseur (7) qu'ils se situent en commun dans la zone focale (F), et que le premier et le second réflecteurs (11, 13) sont situés à des distances inégales du dispositif diviseur (7).

2. Dispositif selon la revendication 1, caractérisé par le fait que l'on prévoit, comme dispositif diviseur (7), un cône fait avec un matériau réfléchissant, distribuant l'onde de choc (5) sur le premier et le second réflecteurs (11, 13).

3. Dispositif selon la revendication 2, caractérisé par le fait que le cône est un cône à 90°, dont le sommet est dirigé vers le tube de choc (1).

4. Dispositif selon la revendication 2 ou 3, caractérisé par le fait que le premier et le second réflecteurs (11, 13) sont chacun une partie d'un anneau qui est formé par la rotation de l'arc d'une première et d'une seconde paraboles autour de l'axe du cône (7), et que les deux réflecteurs (11, 13) sont disposés sur les côtés opposés de l'axe (9a) du cône (7).

5. Dispositif selon l'une des revendications 2 à 4, caractérisé par le fait que le cône (7) est fait avec du laiton.

6. Dispositif selon l'une des revendications 2 à 5, caractérisé par le fait que l'un des deux réflecteurs (11, 13) est échangeable.

7. Dispositif pour produire une onde de choc acoustique et pour la focalisation de celle-ci sur une zone focale, avec un tube de choc unique, caractérisé par le fait que l'onde de choc (5) qui est émise par le tube de choc (1), est subdivisée en plusieurs ondes de choc individuelles (5a, 5b) qui ont des durées de transit différentes.